Europäisches Patentamt

European Patent Office

Office européen des brevets

(11) Veröffentlichungsnummer: **0 260 597 B1**

(12) **EUROPÄISCHE PATENTSCHRIFT**

(45) Veröffentlichungstag der Patentschrift: **28.07.93**

(21) Anmeldenummer: **87113221.3**

(22) Anmeldetag: **10.09.87**

(51) Int. Cl.⁵: **C12N 15/54**, C12P 13/22, C12N 1/20, //(C12N1/20, C12R1:19)

(54) **Klonierung und Verwendung des Transaminase-Gens tyrB.**

(30) Priorität: **19.09.86 DE 3631829**

(43) Veröffentlichungstag der Anmeldung:
**23.03.88 Patentblatt 88/12**

(45) Bekanntmachung des Hinweises auf die Patenterteilung:
**28.07.93 Patentblatt 93/30**

(84) Benannte Vertragsstaaten:
**AT BE CH DE ES FR GB GR IT LI LU NL SE**

(56) Entgegenhaltungen:
**EP-A- 0 116 860**
**EP-A- 0 132 999**
**EP-A- 0 151 488**
**EP-A- 0 152 275**
**EP-A- 0 289 846**

**CHEMICAL ABSTRACTS Band 108, Nr.1, 4. Jan. 1988, S.151, Zusammenfassung Nr. 1525k, Columbus, Ohio, USA; MARTYN ROBINSON et al.:" Cloning of the gene coding for aromatic amino acid aminotransferas from an E coli B strain."**

(73) Patentinhaber: **HOECHST AKTIENGESELL-SCHAFT**
**Postfach 80 03 20**
**W-6230 Frankfurt am Main 80(DE)**

(72) Erfinder: **Marquardt, Rüdiger, Dr.**
**Günthersburgallee 69**
**W-6000 Frankfurt am Main(DE)**
Erfinder: **Then, Johann, Dr.**
**Sulzbacher Weg 2**
**W-6238 Hofheim am Taunus(DE)**
Erfinder: **Deger, Hans-Matthias, Dr.**
**Am Rheingauer Weg 8**
**W-6238 Hofheim am Taunus(DE)**
Erfinder: **Wöhner, Gerhard, Dr.**
**Flörsheimer Strasse 27**
**W-6093 Flörsheim am Main(DE)**
Erfinder: **Robinson, Martyn, Dr.**
**45, Prince Andrew Road**
**Maidenhead Berkshire SL6 8OO(GB)**
Erfinder: **Doherty, Andrew, Dr.**

**Verstorben(GB)**

## Beschreibung

Der letzte Schritt der de novo-Synthese von Phenylalanin besteht in der Aminierung von Phenylpyruvat mit Hilfe einer Transaminase. Obwohl verschiedene Transaminasen in der Lage sind, Phenylpyruvat zu Phenylalanin zu transaminieren, wird diese Funktion in der Zelle hauptsächlich von der sogenannten aromatischen Transaminase erfüllt. Die genetische Abkürzung für das Gen der aromatischen Transaminase in E.coli ist tyrB (Umbarger, Ann. Rev. Biochemistry 47 (1978), 533-606). Im Falle von E. coli K12 ist die Lage des Gens auf dem "Bakterienchromosom" genau bekannt, und das Genprodukt hat die E.C.-Nummer 2.6.1.5 erhalten (Bachmann et. al., Microbiological Reviews 44 (1980), 1-56).

In der Europäischen Patentanmeldung 0 116 860 wird die Isolierung des tyrB-Gens aus E. coli K12 beschrieben sowie die Klonierung dieses Aminotransferase-Gens auf ein Multi-Copy-Plasmid. Das klonierte Plasmid wird wieder zurück in den Stamm gegeben, aus dem das Gen urspünglich isoliert worden war, mit dem Ergebnis, daß die L-Phenylalanin-Produktion in diesem Stamm um etwa 11 % erhöht werden konnte.

Es wurde nun überraschend gefunden, daß die Isolierung des in E. coli ATCC 11303 vorhandenen tyrB-Gens und dessen Klonierung auf ein Multi-Copy-Plasmid nach der Transformation von Mikroorganismen mit diesem Plasmid, insbesondere des Ausgangsstamms, in einer 10fachen Steigerung der L-Phenylalaninausbeute resultiert.

Die Erfindung betrifft somit:

1. Ein replizierendes extra-chromosomales Element, welches das aus E. coli ATCC 11303 isolierte tyrB-Gen enthält.

2. Die Verwendung des unter 1) genannten extra-chromosomalen Elements zur Synthese der aromatischen Aminotransferase.

3. Die Verwendung des unter 1) genannten extra-chromosomalen Elements zur Überproduktion von L-Phenylalanin in Mikroorganismen, die dadurch gekennzeichnet ist, daß

    a) das extra-chromosomale Element in einen Mikroorganismus eingebracht wird,

    b) das tyrB-Gen in deisem Mikroorganismus exprimiert wird und eine aktive aromatische Transaminase synthetisiert und

    c) die Transaminase die Aminierung von Phenylpyruvat bewirkt.

Die Erfindung wird in der folgenden Beschreibung detailliert erläutert bzw. in den Patentansprüchen definiert.

Außer dem Wildtyp E. coli ATCC 11303 können auch dessen Varianten und Mutanten verwendet werden. Beispielsweise kann auch ein nach bekannten Methoden mutierter Stamm [E. Adelberg et al., Biochem. Biophys. Res. Comm. 18, 788 (1965)], der auf Überproduktion von L-Phenylalanin selektioniert wurde, eingesetzt werden. Die aromatische Aminotransferase aus E. coli ATCC 11303, für die das tyrB-Gen codiert, synthesiert unter anderem L-Phenylalanin aus Phenylpyruvat, indem eine Aminogruppe aus Glutamat transferiert wird. Mit Hilfe der aromatischen Transaminase können außerdem Tyrosin, Glutamat, Aspartat und Leucin synthetisiert werden. Die Aminosäuren Isoleucin, Valin, Leucin, Phenylalanin und Glutaminsäure werden mit Hilfe einer Transaminase gebildet, für die das Gen ilvE codiert, während die Aminosäuren Aspartat, Glutamat, Phenylalanin und Tyrosin von einer Transaminase synthetisiert werden, für deren Expression das aspC-Gen verantwortlich ist. Allerdings zeigt jede der genannten Transaminasen auch eine schwache Aktivität bei der Synthese von Aminosäuren, die eigentlich spezifischer einer der beiden anderen Transaminasen zuzuordnen sind.

Um die Synthese von L-Phenylalanin noch weiter zu erhöhen, wird das tyrB-Gen, das für die aromatische Aminotransferase codiert, kloniert. Man erreicht dies durch Isolierung der DNA aus E. coli ATCC 11303. Nach partieller Verdauung der DNA werden die entstandenen Fragmente, die sich in einem Größenbereich von 20-30 kb bewegen, in ein Cosmid mit einem Replikon, das einen weiten Wirtsbereich vermittelt, ligiert und in die Köpfe des λ-Phagen verpackt. Bevorzugt wird das Cosmid pIMS 6026 verwendet. Das Cosmid pIMS 6026 leitet sich von dem Cosmid pLAFR 1 (ATCC 37167) dadurch ab, daß in die einzige EcoRI Schnittstelle des Cosmids pLAFR 1 das handelsübliche EcoRI Fragment (Pharmacia, Upsala, Schweden) kloniert wurde, auf dem das Kanamycin-Resistenzgen des Transposons Tn 903 liegt. Durch Verdauung mit Bam HI und anschließende Religation kann der größte Teil des handelsüblichen Eco RI-Fragments deletiert werden, so daß ein kurzes Stück DNA als Insertion zurückbleibt, in der eine BamHI-Schnittstelle von 2 Eco RI-Schnittstellen flankiert wird. Diese BamHI-Schnittstelle, die auf dem Cosmid pLAFR 1 nicht vorhanden ist, kann für Klonierungen verwendet werden. Durch Inkubation der verpackten Cosmide mit einer entsprechend vorbereiteten E. coli DG 30-Suspension werden die Cosmide in den Mikroorganismus eingeschleust. E. coli DG 30 hat eine Defizienz in den drei Transaminasen aspC, ilvE und tyrB. Der Stamm wächst daher auf Vollmedium zwar problemlos an, muß aber bei Wachstum auf Minimalmedium mit verschiedenen Aminosäuren von außen versorgt werden, da er sie nicht selbst synthetisieren kann. Bei geeigneter Wahl des Mediums kann mit Hilfe des Stamms untersucht werden, ob eine von außen aufgenommene DNA den chromosomalen Defekt für eine bestimmte Transaminase komplementieren kann. Die Einschleusung des tyrB-Gens detektiert man durch Wachstum des E. coli DG 30 auf einem tyrosinfreien Minimalmedium. Auf diesem Medium können nur Klone anwachsen, die durch Aufnahme von aspC, tyrB oder ilvE enthaltender DNA, die ursprünglich aus dem Stamm E. coli ATCC 11303 stammt, ihren chromosomalen Defekt für die Synthese von Tyrosin komplementieren können. Die drei Transaminasen, die von den Genen aspC, tyrB und ilvE codiert werden, unterscheiden sich in ihrer Substratspezifität, wenngleich sie alle drei in der Lage sind, Tyrosin zu bilden.

Die aromatische Transaminase, die von dem Gen tyrB codiert wird, kann beispielsweise kein Isoleucin aus der Ketovorstufe bilden, wohl aber Leucin in guten Ausbeuten aus der entsprechenden Ketovorstufe synthetisieren. Die Transaminase, die von dem Gen aspC codiert wird, kann weder Isoleucin bilden noch ist sie effizient in der Umsetzung zu Leucin.

Dementsprechend können die einzelnen Klone durch Wachstum auf einem Minimalmedium, das bis auf eine für das jeweilige Gen charakteristische Aminosäure mit für den Stoffwechsel essentiellen Aminosäuren supplementiert ist, hinsichtlich der enthaltenen Transaminase unterschieden werden. Auf diese Weise werden Klone des DG-30 Stammes, die das tyrB-Gen enthalten, heraus selektioniert.

Es muß nun überprüft werden, ob die Klone auch wirklich das tyrB-Gen besitzen. Hierzu muß die Plasmid-DNA aus den Klonen isoliert werden. Die Isolierung aus dem Stamm DG 30 gelingt jedoch nur mit Schwierigkeiten. Es kann zwar Plasmid-DNA erhalten werden, jedoch nur in geringen Ausbeuten. Nach Minilyse wird daher ein E. coli-Stamm mit den Cosmid-DNAs aus den interessanten Klonen transformiert, aus der anschließend die eingeschleuste DNA in guten Ausbeuten reisoliert werden kann. E. coli DH1 (ATCC 33849) ist hierzu insbesondere geeignet.

In dem nächsten Schritt wird die reisolierte Cosmid DNA mit einem Vektor hoher Kopienzahl ligiert. Aus der chromosomalen Genkarte des Stammes E. coli K12 ist bekannt, daß das tyrB-Gen auf einem ClaI Fragment lokalisiert ist. Es bestand die Möglichkeit, daß dies auch bei E. coli ATCC 11303 der Fall ist. Aus diesem Grund wird als Vektor bevorzugt ein Multi-Copy-Plasmid mit einer ClaI-Schnittstelle eingesetzt, insbesondere bevorzugt das in seiner Sequenz bekannte pAT 153 (Winnacker E.L., Gene und Klone, VCH-Verlagsgesellschaft, Weinheim).

Die Cosmid-DNA und der Vektor werden mit dem Restriktionsenzym ClaI vollständig verdaut. Die beiden DNAs werden zusammengegeben, miteinander ligiert un kompetente Zellen eines Wirtsorganismus, in dem die Phenylalaninproduktion erhöht werden soll, damit transformiert. Bevorzugt werden Enterobakterien eingesetzt, insbesondere E. coli, wobei E. coli ATCC 11303 sowie seine Mutanten und Varianten besonders bevorzugt sind. Resistente Kolonien werden über Ampicillin selektioniert und anhand von Marker-Inhibierung durch "replica plating" auf Tetracyclin-Platten auf Einbau getestet. Aus den entsprechenden Kolonien wird durch Minilyse Plasmid-DNA isoliert und durch vollständige Verdauung mit dem Restriktionsenzym ClaI das Vorhandensein von ClaI-Fragmenten in dem Vektor überprüft. Durch Restriktionsanalyse wird sichergestellt, daß alle in dem ursprünglichen DNA-Abschnitt enthaltenen ClaI-Fragmente auf diese Art subkloniert worden sind. Klone, die jeweils eins dieser definierten ClaI-Fragmente enthalten, werden schließlich mittels Aspartat-Phenylpyruvat-Aminotransferase (APPAT)-Test auf die Aktivität der aromatischen Transaminase, also des Genprodukts von tyrB, getestet. Auf diese Weise läßt sich eine Zunahme der tyrB-Aktivität um den Faktor 5 bis 10 erreichen. Durch Agarose-Gelelektrophorese kann gezeigt werden, daß in dem Vektor des Wirtsstammes ein ca. 2,7 MD großes ClaI-Fragment enthalten ist.

In den folgenden Beispielen wird die Erfindung im einzelnen beschrieben. Prozentangaben beziehen sich, wenn nicht anders angegeben, auf das Gewicht.

Beispiel 1

Isolierung und Verdauung des Cosmids pIMS 6026 aus E. coli

Für die Isolierung des Cosmids pIMS 6026 aus E. coli wurde entweder nach Humphreys et al. vorgegangen [Biochem. Biophys. Acta 383, 457-63 (1975)] oder eine alkalische Lyse nach Birnboim und Doly [Nucleic Acids Res. 7 : 151 ] im 10-fach größeren Maßstab durchgeführt. In jedem Fall wurde die Plasmid-DNA mindestens einmal durch CsCl/EtBr-Dichtegradientenzentrifugation gereinigt.

Das Cosmid pIMS 6026 wurde vollständig mit dem Restriktionsenzym BamHI verdaut, wobei nach den Angaben des Herstellers, New England Biolabs, verfahren wurde. Zur Überprüfung der Vollständigkeit dieser Verdauung wurde ein Aliquot des Restriktionsansatzes auf ein 0,8 %iges Agarosegel aufgetragen und der Elektrophorese unterworfen. Das Erscheinen nur einer Bande nach Anfärben mit Ethidiumbromid und Bestrahlung mit kurzwelligem UV-Licht (254nm) diente als Hinweis auf eine vollständige Verdauung. Von der verdauten Cosmid-DNA wurde das Restriktionsenzym durch Phenolisierung entfernt, die DNA mittels Ethanol gefällt, mit 70 %igem Ethanol gewaschen und nach Trocknen unter Vakuum in einem geeigneten Volumen an TE-Puffer(10 mM Tris; 1 mM EDTA, pH 8,0) aufgenommen. Wahlweise wurde noch eine Behandlung mit alkalischer Phosphatase nach den Angaben des Herstellers Boehringer Mannheim, durchgeführt. Nach Zugabe von 1 $\mu$l an alkalischer Phosphatase (CIP) wurde 30 Minuten lang bei 37°C inkubiert, das Enzym durch Phenolisierung aus dem Reaktionsansatz entfernt und die DNA, wie oben beschrieben, gereinigt. Schließlich wurde sie in TE-Puffer resuspendiert.

Beispiel 2

Partielle Verdauung der DNA aus E. coli ATCC 11303

Die Isolierung der Gesamt-DNA aus E. coli ATCC 11303 wurde nach der Methode von Marmur in J. Mol. Biol. 53, 155-162, (1961) durchgeführt.Die isolierte Gesamt-DNA wurde mit dem Restriktionsenzym Sau3A partiell verdaut, so daß hauptsächlich Fragmente in einem Größenbereich von 20-30kb entstanden. Dazu wurde in Vorversuchen das hierfür optimale Verhältnis von DNA und Enzym sowie die optimale Einwirkdauer des Enzyms auf die DNA festgestellt. Die entsprechende Vorgehensweise ist in dem von der Firma BRL herausgegebenen Heft "focus", Vol. 7, Nr 2 (1985) auf Seite 3 beschrieben. Nach Ablauf der als optimal bestimmten Reaktionszeit wurde das Enzym durch Erhitzen auf 65°C über einen Zeitraum von 10 Minuten zerstört und die Bildung von DNA-Fragmenten im gewünschten Größenbereich durch Agarose-Gelelektrophorese mit geeigneten DNA-Markern, z.B. mit EcoRI-verdauter DNA des Phagen $\lambda$, überprüft.

Beispiel 3

Ligation der Restriktionsansätze

Mit Sau3A partiell verdaute Gesamt-DNA aus E. coli ATCC 11303 wurde mit pIMS 6026 Cosmid-DNA, die mit BamHI vollständig gespalten und mit alkalischer Phosphatase behandelt worden war, in einem molaren Verhältnis von etwa 1 : 5 zusammengegeben. Die resultierende Mischung wurde mit einem mehrfach konzentrierten Puffer nach den Angaben von New England Biolabs so versetzt, daß eine für das Enzym T4-DNA-Ligase optimale Ionenkonzentration resultierte, und mit 1 $\mu$l des Enzyms mindestens 14 Stunden lang bei 16°C inkubiert. Das Gesamtvolumen des Ansatzes betrug dabei 50 $\mu$l mit einer Gesamt-DNA-Konzentration von 20 $\mu$g/ml.

Beispiel 4

Verpackung in $\lambda$-Phagen

Nach erfolgter Ligase-Reaktion wurde nach Beispiel 3 erhaltene DNA in vitro in die Köpfe von $\lambda$-Phagen verpackt. Die dafür notwendigen Extrakte aus zwei verschiedenen Bakterienstämmen können nach Hohn, B., in Wu,R., editor: Recombinant DNA, Methods in Enzymology, Vol. 68, Academic Press, New York, S.299-309 (1979) gewonnen werden oder von Boehringer/Mannheim oder Amersham Buchler, Braunschweig, bezogen werden.
3$\mu$l der nach Beispiel 3 erhaltenen Mischung wurden mit unmittelbar zuvor aufgetauten Bakterien-Extrakten der Firma Amersham unter Eiskühlung gründlich vermischt. Die Mischung wurde 30-60 Minuten bei 20°C inkubiert und anschließend 200$\mu$l SM-Puffer (100 mM NaCl, 10 mM MgSO$_4$, 50 mM Tris-HCl (pH 7,5), 0,01 % Gelatine) zugegeben. Diese Mischung wurde entweder direkt in eine Transduktions-Reaktion eingesetzt oder nach Zugabe von 10 $\mu$l Chloroform bis zur späteren Verwendung bei 4°C aufbewahrt.

Beispiel 5

Transduktion von E. coli DG 30

5 ml L-Broth, bestehend aus 1 % Bacto-Trypton, 0,5 % Hefeextrakt und 0,5 % NaCl, wurden 0,4 % Maltose zugesetzt und mit 50 $\mu$l einer Flüssigkultur von E. coli DG 30 in der stationären Wachstumsphase beimpft. Es wurde 12 Stunden bei 37°C inkubiert, bis die frühe stationäre Phase erreicht war. Die Bakterien wurden abzentrifugiert und vorsichtig in 2,5 ml einer wäßrigen Lösung, die 10 mmolar an MgCl$_2$ war, resuspendiert. 10 $\mu$l der Mischung gemäß Beispiel 4 wurden mit 20 $\mu$l der konzentrierten Bakteriensuspen-

sion versetzt und 50 Minuten bei Raumtemperatur inkubiert.

Anschließend wurde 200 $\mu$l L-Broth hizugegeben und 1 Stunde bei 37°C inkubiert, wobei die Mischung gelegentlich geschüttelt wurde.

Jeweils 50 $\mu$l des Ansatzes wurden auf L-Broth-Agar, der 20 $\mu$g/ml Tetracyclin enthielt, ausplattiert. Die Platten wurden mindestens 12 Stunden bei 37°C bebrütet. Bei der beschriebenen Vorgehensweise konnten aus einem Ansatz durchschnittlich 1000 Kolonien erhalten werden.

Beispiel 6

Selektionierung von E. coli DG 30 mit einem aspC- oder ilvE- oder tyrB-Gen.

Rund 800 Kolonien, die nach Transduktion von E. coli DG 30 nach dem beschriebenen Verfahren auf L-Broth-Agar, der 20 $\mu$g/ml Tetracyclin enthielt, erhalten worden waren, wurden auf Minimalagar "gepickt". Der Minimalagar bestand aus M9-Medium mit Glukose (Miller, Experiments in Molecular Genetics, Cold Spring Harbor, 1972), das durch die Aminosäuren Isoleucin, Leucin, Valin, Asparaginsäure und Phenylalanin supplementiert worden war. Die Aminosäure Tyrosin, die der Stamm DG 30 gleichfalls nicht mehr synthetisieren kann, wurde dem Medium jedoch nicht zugegeben. Von den 800 "gepickten" Kolonien konnten 7 auf dem Minimalmedium anwachsen.

Zur Unterscheidung der drei möglichen Gene aspC, ilvE und tyrB in E. coli DG 30 wurden diese 7 Kolonien wiederum auf oben genanntes Minimalmedium "gepickt", das mit den aufgeführten Aminosäuren supplementiert war, bis auf jeweils eine, für die eine der Transaminasen, die von einem der Gene codiert wird, Substratspezifität zeigt. Das Ergebnis ist in der nachstehenden Tabelle aufgeführt:

| Klon | Minimalmedium, supplementiert bis auf | | | | vermutliches |
|------|-----|-----|-----|-----|--------------|
|      | Asp | Leu | Ile | tyr | Gen |
| 1 | + | + | – | + | tyrB |
| 2 | + | + | – | + | tyrB |
| 3 | – | +– | + | +– | ilvE |
| 4 | – | +– | + | +– | ilvE |
| 5 | + | + | – | + | tyrB |
| 6 | + | + | – | + | tyrB |
| 7 | – | +– | + | +– | ilvE |

+ = gutes Wachstum

+– = schlechtes Wachstum

– = kein Wachstum

Beispiel 7

Lokalisierung des tyrB-Gens

Durch Minilyse nach Maniatis et al., Cold Spring Harbor, Seiten 366-370 (1982), wurde Cosmid-DNA der Klone 1 bis 7, die gemäß Beispiel 6 erhalten wurden, gewonnen.

Anschließend wurde diese Cosmid-DNA in E. coli DH1 (ATCC 33849) eingeschleust, woraus sie in guten Ausbeuten wieder reisoliert werden konnte.

Es wurde Plasmid-DNA, die ursprünglich aus dem Klon 5 von E. coli DG 30 (siehe Beispiel 6) gewonnen wurde, aus dem mit dieser DNA transformierten Stamm E. coli DH1 isoliert und mit dem Restriktionsenzym ClaI, den Angaben des Herstellers, New England Biolabs, folgend, vollständig verdaut. Gleichfalls vollständig mit ClaI verdaut wurde der Vektor pAT 153, der anschließend noch einer Behandlung mit alkalischer Phosphatase unterzogen wurde. Die beiden DNAs wurden zusammengegeben, nach der in Beispiel 4 bereits beschriebenen Art und Weise miteinander ligiert und kompetente Zellen des Stammes E. coli ATCC 11303 mit einem Aliquot des Ligase-Ansatzes, z.B. 10 $\mu$l, transformiert. Resistente Kolonien wurden auf L-Broth-Platten, die 50 $\mu$g/ml Ampicillin enthielten, selektioniert und durch "replica plating" auf L-Broth-Platten mit 20 $\mu$g/ml Tetracyclin auf Markerinaktivierung und damit Einbau getestet. Aus Kolonien, die den Phänotyp Ap$^r$Tc$^s$ aufwiesen, wurde durch Minilyse Plasmid-DNA isoliert und durch vollständige Verdauung mit dem Restriktionsenzym ClaI das Vorhandensein von ClaI-Fragmenten in dem Vektor pAT153 überprüft.

Beispiel 8

Überprüfung der Transaminase-Aktivität

Die gemäß Beispiel 7 erhaltenen Klone wurden mittels APPAT-Test (Aspartat-Phenylpyruvat-Aminotransferase Assay, Sigma Testkit G0390, wobei $\alpha$-Ketoglutarat durch Phenylpyruvat ersetzt wurde) auf die Aktivität der aromatischen Transaminase, also des Genprodukts von tyrB, getestet. Als Vegleich diente der nicht transformierte Ausgangsstamm E. coli ATCC 11303. Dabei konnte in einem Fall eine deutliche Zunahme an tyrB-Aktivität, und zwar um den Faktor 5 bis 10, gegenüber dem Ausgangsstamm E. coli ATCC 11303, gemessen werden.

Durch Agarose-Gelelektrophorese unter Verwendung geeigneter Marker konnte gezeigt werden, daß in dem Stamm, der erhöhte tyrB-Genaktivität aufwies, ein pAT 153-Vektor enthalten war, der ein rund 2,7 MD großes ClaI-Fragment eingebaut enthielt. Wurde mit der isolierten Plasmid-DNA erneut der plasmidlose Stamm E. coli ATCC 11303 transformiert, so konnte in jedem Fall eine Zunahme der tryB-Genaktivität um den Faktor 5-10 beobachtet werden. Das entsprechende Plasmid erhielt die Bezeichnung pIMS 6056.

**Patentansprüche**
**Patentansprüche für folgende Vertragsstaaten : BE, CH, DE, FR, GB, IT, LI, LU, NL, SE**

1.  Ein replizierendes extra-chromosomales Element, welches das aus E. coli ATCC 11303 isolierte tyrB-Gen enthält.

2.  Das extra-chromosomale Element nach Anspruch 1, dadurch gekennzeichnet, daß es ein Multi-Copy-Plasmid ist.

3.  Das extra-chromosomale Element nach Anspruch 2, dadurch gekennzeichnet, daß es das Multi-Copy-Plasmid pAT 153 ist.

4.  Verwendung des extra-chromosomalen Elements nach einem oder mehreren der Ansprüche 1 bis 3 zur Synthese der aromatischen Transaminase.

5.  Verwendung des extra-chromosomalen Elements nach einem oder mehreren der Ansprüche 1 bis 3 zur Überproduktion von L-Phenylalanin in Mikroorganismen, dadurch gekennzeichnet, daß
    a) das extra-chromosomale Element in einen Mikroorganismus eingebracht wird,
    b) das tyrB-Gen in dem Mikroorganismus exprimiert wird und eine aktive aromatische Transaminase synthetisiert und
    c) die Transaminase die Aminierung von Phenylpyruvat bewirkt.

6.  Verwendung nach Anspruch 4, dadurch gekennzeichnet, daß der Mikroorganismus ein Enterobakterium ist.

7.  Verwendung nach Anspruch 5, dadurch gekennzeichnet, daß der Mikroorganismus E. coli ist.

8.  Verwendung nach Anspruch 7, dadurch gekennzeichnet, daß der Mikroorganismus E. coli ATCC 11303 ist sowie dessen Varianten und Mutanten, soweit sie die gleiche Funktion hinsichtlich der Phenylalaninproduktion wie E. coli ATCC 11303 besitzen.

9.  E. coli ATCC 11303 sowie dessen Varianten und Mutanten, soweit sie die gleiche Funktion hinsichtlich der Phenylalaninproduktion wie E. coli ATCC 11303 besitzen, transformiert mit dem extra-chromosomalen Element nach einem oder mehreren der Ansprüche 1 bis 3.

**10.** TyrB-Gen, erhältlich aus E. coli ATCC 11303 sowie dessen Varianten und Mutanten, soweit sie die gleiche Funktion hinsichtlich der Phenylalaninproduktion wie E. coli ATCC 11303 besitzen und aus ihnen das gleiche Element wie das nach Anspruch 1 herstellbar ist.

**Patentansprüche für folgende Vertragsstaaten : AT, ES, GR**

**1.** Verfahren zur Herstellung eines ein tyrB-Gen enthaltenden replizierenden extrachromosomalen Elements, dadurch gekennzeichnet, daß das tyrB-Gen aus E. coli ATCC 11 303 isoliert und in ein Plasmid cloniert wird.

**2.** Verfahren nach Anspruch 1, dadurch gekennzeichnet, daß als Plasmid ein Multi-Copy-Plasmid eingesetzt wird.

**3.** Verfahren nach Anspruch 2, dadurch gekennzeichnet, daß als Plasmid pAT 153 eingesetzt wird.

**4.** Verfahren zur Überproduktion von L-Phenylalanin in Mikroorganismen, dadurch gekennzeichnet, daß
   a) das extra-chromosomale Element, erhältlich nach Anspruch 1, in einen Mikroorganismus eingebracht wird,
   b) das tyrB-Gen in dem Mikroorganismus exprimiert wird und eine aktive aromatische Transaminase synthetisiert und
   c) die Transaminase die Aminierung von Phenylpyruvat bewirkt.

**5.** Verfahren nach Anspruch 4, dadurch gekennzeichnet, daß der Mikroorganismus ein Enterobakterium ist.

**6.** Verfahren nach Anspruch 5, dadurch gekennzeichnet, daß der Mikroorganismus E. coli ist.

**7.** Verfahren nach Anspruch 6, dadurch gekennzeichnet, daß der Mikroorganismus E. coli ATCC 11303 ist sowie dessen Varianten und Mutanten soweit sie die gleiche Funktion hinsichtlich der Phenylalaninproduktion wie E. coli ATCC 11303 besitzen und aus ihnen das gleiche Element wie das nach Anspruch 1 herstellbar ist.

**Claims**
**Claims for the following Contracting States : BE, CH, DE, FR, GB, IT, LI, LU, NL, SE**

**1.** A replicating extrachromosomal element which contains the tyrB gene isolated from E. coli ATCC 11303.

**2.** The extrachromosomal element as claimed in claim 1, which is a multicopy plasmid.

**3.** The extrachromosomal element as claimed in claim 2, wherein the multicopy plasmid is pAT 153.

**4.** The use of the extrachromosomal element as claimed in one or more of claims 1 to 3 for the synthesis of aromatic transaminase.

**5.** The use of the extrachromosomal element as claimed in one or more of claims 1 to 3 for the overproduction of L-phenylalanine in microorganisms, which comprises
   a) introduction of the extrachromosomal element into a microorganism,
   b) expression of the tyrB gene in the microorganism, and synthesis of an active aromatic transaminase, and
   c) amination of phenylpyruvate by the transaminase.

**6.** The use as claimed in claim 4, wherein the microorganism is an enterobacterium.

**7.** The use as claimed in claim 5, wherein the microorganism is E. coli.

**8.** The use as claimed in claim 7, wherein the microorganism is E. coli ATCC 11303 and its variants and mutants, insofar as they have the same function with respect to phenylalanine production as E. coli ATCC 11303.

**9.** E. coli ATCC 11303, and its variants and mutants, insofar as they have the same function with respect to phenylalanine production as E. coli ATCC 11303, transformed with the extrachromasomal element as claimed in one or more of claims 1 to 3.

**10.** The tyrB gene obtainable from E. coli ATCC 11303 and its variants and mutants, insofar as they have the same function with respect to phenylalanine production as E. coli ATCC 11303 and the same element as that claimed in claim 1 can be prepared from it.

**Claims for the following Contracting States : AT, ES, GR**

**1.** A process for the preparation of a replicating extrachromosomal element containing a tyrB gene, wherein the tyrB gene is isolated from E. coli ATCC 11303 and cloned in a plasmid.

**2.** The process as claimed in claim 1, wherein the plasmid used is a multicopy plasmid.

**3.** The process as claimed in claim 2, wherein the plasmid used is pAT 153.

**4.** A process for overproduction of L-phenylalanine in microorganisms, which comprises
a) introduction of the extrachromosomal element, obtainable as claimed in claim 1, into a microorganism,
b) expression of the tyrB gene in the microorganism, and synthesis of an active aromatic transaminase, and
c) amination of phenylpyruvate by the transaminase.

**5.** The process as claimed in claim 4, wherein the microorganism is an enterobacterium.

**6.** The process as claimed in claim 5, wherein the microorganism is E. coli.

**7.** The process as claimed in claim 6, wherein the microorganism is E. coli ATCC 11303 and its variants and mutants, insofar as they have the same function with respect to phenylalanine production as E. coli ATCC 11303 and the same element as that claimed in claim 1 can be prepared from it.

**Revendications**
**Revendications pour les Etats contractants suivants : BE, CH, DE, FR, GB, IT, LI, LU, NL, SE**

**1.** Elément extrachromosomique répliquant, qui contient le gène tyrB isolé à partir de *E. coli* ATCC 11303.

**2.** Elément extrachromosomique selon la revendication 1, caractérisé en ce qu'il est un plasmide multicopie.

**3.** Elément extrachromosomique selon la revendication 2, caractérisé en ce que le plasmide multicopie est pAT 153.

**4.** Utilisation de l'élément extrachromosomique selon une ou plusieurs des revendications 1 à 3, pour la synthèse de la transaminase aromatique.

**5.** Utilisation de l'élément extrachromosomique selon une ou plusieurs des revendications 1 à 3, pour la surproduction de L-phénylalanine dans des micro-organismes, caractérisée en ce que
a) on introduit l'élément extrachromosomique dans un micro-organisme,
b) le gène tyrB est exprimé dans le micro-organisme et une transaminase aromatique active est synthétisée, et
c) la transaminase provoque l'amination du phénylpyruvate.

**6.** Utilisation selon la revendication 4, caractérisée en ce que le micro-organisme est une entérobactérie.

**7.** Utilisation selon la revendication 5, caractérisée en ce que le micro-organisme est *E. coli*.

**8.** Utilisation selon la revendication 7, caractérisée en ce que le micro-organisme est *E. coli* ATCC 11303, ainsi que ses variants et mutants, dans la mesure où ils possèdent la même fonction que *E. coli* ATCC 11303 en ce qui concerne la production de phénylalanine.

**9.** *E. coli* ATCC 11303 ainsi que ses variants et mutants, dans la mesure où ils possèdent la même fonction que *E. coli* ATCC 11303 en ce qui concerne la production de phénylalanine, transformés par l'élément extrachromosomique selon une ou plusieurs des revendications 1 à 3.

**10.** Gène tyrB, pouvant être obtenu à partir de *E. coli* ATCC 11303 ainsi que ses variants et mutants, dans la mesure où ils possèdent la même fonction que *E. coli* ATCC 11303 en ce qui concerne la production de phénylalanine, et le même élément que celui selon la revendication 1 peut être produit à partir de ceux-ci.

**Revendications pour les Etats contractants suivants : AT, ES, GR**

**1.** Procédé pour la production d'un élément extrachromosomique répliquant, contenant le gène tyrB, caractérisé en ce que le gène tyrB est isolé à partir de *E. coli* ATCC 11303 et est cloné dans un plasmide.

**2.** Procédé selon la revendication 1, caractérisé en ce que, en tant que plasmide, on utilise un plasmide multicopie.

**3.** Procédé selon la revendication 2, caractérisé en ce que, en tant que plasmide, on utilise pAT 153.

**4.** Procédé pour la surproduction de L-phénylalanine dans des micro-organismes, caractérisé en ce que
a) on introduit dans un microorganisme l'élément extrachromosomique pouvant être obtenu selon la revendication 1,
b) le gène tyrB est exprimé dans le micro-organisme et une transaminase aromatique active est synthétisée, et
c) la transaminase provoque l'amination du phénylpyruvate.

**5.** Procédé selon la revendication 4, caractérisé en ce que le micro-organisme est une entérobactérie.

**6.** Procédé selon la revendication 5, caractérisé en ce que le micro-organisme est *E. coli*.

**7.** Procédé selon la revendication 6, caractérisé en ce que le micro-organisme est *E. coli* ATCC 11303, ainsi que ses variants et mutants, dans la mesure où ils possèdent la même fonction que *E. coli* ATCC 11303 en ce qui concerne la production de phénylalanine, et le même élément que celui selon la revendication 1 peut être obtenu à partir de ceux-ci.

9